# EUROPEAN PATENT SPECIFICATION

(11) **EP 2 457 544 B1**
(45) Date of publication and mention of the grant of the patent: **25.03.2015**
(21) Application number: 11190484.3
(22) Date of filing: 24.11.2011
(51) Int. Cl.: A61F 5/02, A43C 11/14, A61F 5/30

(54) **Improved hyperextension orthopaedic corset**
Verbessertes orthopädisches Hyperextensionskorsett
Corset orthopédique d'hyperextension amélioré

(30) Priority: 25.11.2010 IT MI20102189
(43) Date of publication of application: 30.05.2012
(73) Proprietor: Orthoservice AG, 6830 Chiasso (CH)
(72) Inventor: Bernareggi, Aldo, 20842 Besana Brianza (MB) (IT)
(74) Representative: Ottazzo, Marco Francesco Agostino

(56) References cited:
- EP-A1- 1 269 942
- WO-A1-2006/095162
- US-A- 3 220 407
- US-A1- 2002 178 548
- US-B1- 6 471 665

## Description

The present invention refers to an improved hyperextension orthopaedic corset, that can be used for the treatment of mildly serious and serious dorso-lumbar pathologies.

Numerous pathologies or results from trauma require the stabilization of the spinal column and an anti-gravitational action on the rachis, which permits to lighten it from the load of the upper part of the trunk that normally, by gravity, indeed discharges along the rachis.

The pathologies of interest are the results from traumas, such as spinal fractures, tumour pathologies, which can lead to spinal metastasis with consequent increased porosity of the affected bone structure, or osteoporosis due to aging, which can lead to vertebral collapses associated with compression of the nerve endings.

Currently, the needs for immobilisation and lightening of the load on the rachis are solved by using a so-called hyperextension corset, which the patient must always wear when he is in the erect position. In such a circumstance, indeed, the load of the upper part of the trunk entirely weighs down upon the spinal column.

The hyperextension orthopaedic corset consists of a frame structure in light aluminium alloy which, once worn on the trunk, offers two thrust points in the front part, that can be identified in the chest area (breastbone) and on the pelvis, and a thrust point in the rear part (indicatively at the dorso-lumbar transition), created with a rigid belt constrained to the aluminium structure.

In order to improve the comfort of patients, the supports points on the chest and on the lumbar area consist of stuffed plates, which ensure the advantageous distribution of the pressure on a more extended body surface.

Hyperextension orthopaedic corsets are normally available in different sizes, but must in any case be perfectly adjusted and adapted on the basis of the morphology of each single patient. The adjustments may concern the width of the chest at the height of the breastbone, the height of the trunk and the inclination of the pelvic strap. In order to wear the hyperextension corset, in addition to carrying out the adjustments of the frame structure through screws, it is necessary to arrange the lumbar plate pulling tight the rear belt through a system of buckles, which do not always allow the orthopaedics technician to perform a stable and precise adjustment.

Also the pelvic strap, which in some cases must vary its actual relative position with respect to the frame structure, for example to allow the patient to sit while wearing the corset, is adjusted through a screw system. It is therefore necessary for the orthopaedics technician to have considerable manual ability in order to position and lock the screws, bearing also in mind that the patient generally has limited mobility and can also suffer pain during the adjustment steps. The risk of losing or damaging the adjustment screws can also be high.

Finally, with the current adjustment systems misalignments can occur between the components of the hyperextension corset, especially during the adjustment steps of the relative position of the pelvic strap with respect to the frame structure. These misalignments can lead to deformations on the components of the hyperextension corset which, although of limited extent, can jeopardise the functionality and the stability of the hyperextension corset itself, as well as the level of comfort of the patient.

Document WO 2006/095162 A1 discloses a hyperextension orthopaedic corset according to the preamble of claim 1. Such a hyperextension corset is provided with an adjustment system of known type. Document US 3 220 407 A discloses another type of hyperextension orthopaedic corset of known type. Finally, document US 2002/178548 A1 discloses a closure system provided with toothed means.

The purpose of the present invention is therefore that of realizing an improved hyperextension orthopaedic corset which can be precisely and safely adjusted, combining the needs both of the patient and of the orthopaedics technician.

Another purpose of the invention is that of realizing an improved hyperextension orthopaedic corset in which deformations occurred during the adjustment steps of the various components are avoided, or in any case minimised.

A further purpose of the present invention is that of realizing an improved hyperextension orthopaedic corset that is particularly simple and functional, with low costs.

These purposes according to the present invention are achieved by realizing an improved hyperextension orthopaedic corset as outlined in claim 1.

Further characteristics of the invention are highlighted by the dependent claims, which are an integrating part of the present description.

The characteristics and advantages of an improved hyperextension orthopaedic corset according to the present invention shall become clearer from the following description, exemplifying and not limiting, with reference to the attached schematic drawings, in wherein:

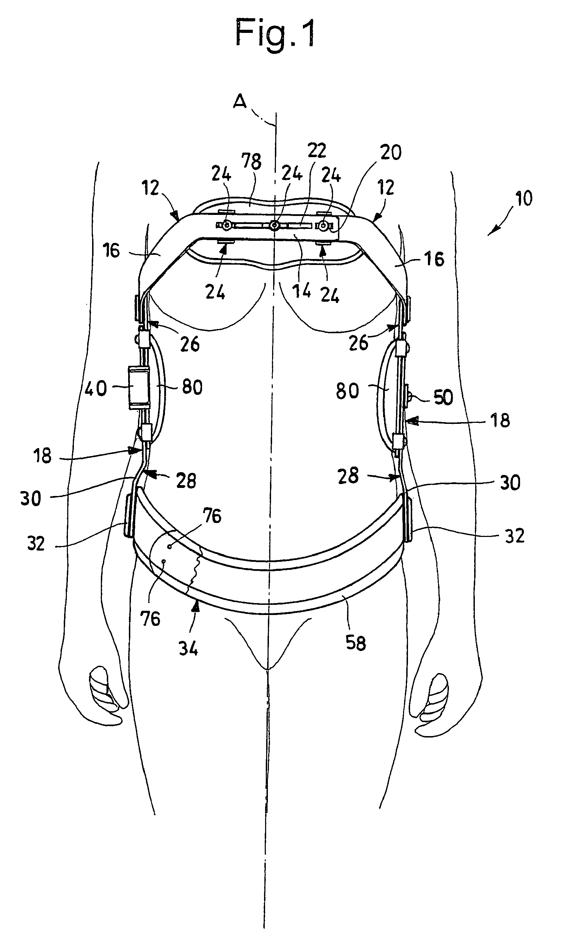

figure 1 is a front view of a hyperextension orthopaedic corset being worn;

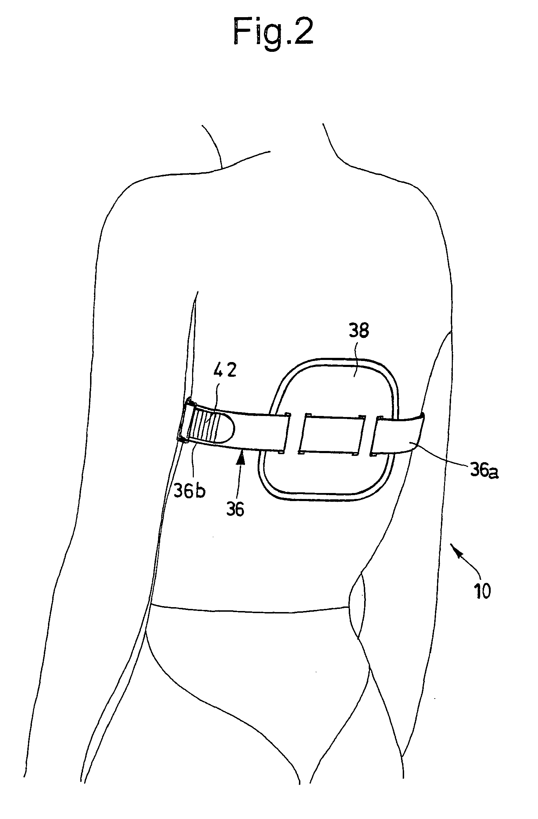

figure 2 is a rear view of the hyperextension orthopaedic corset of figure 1;

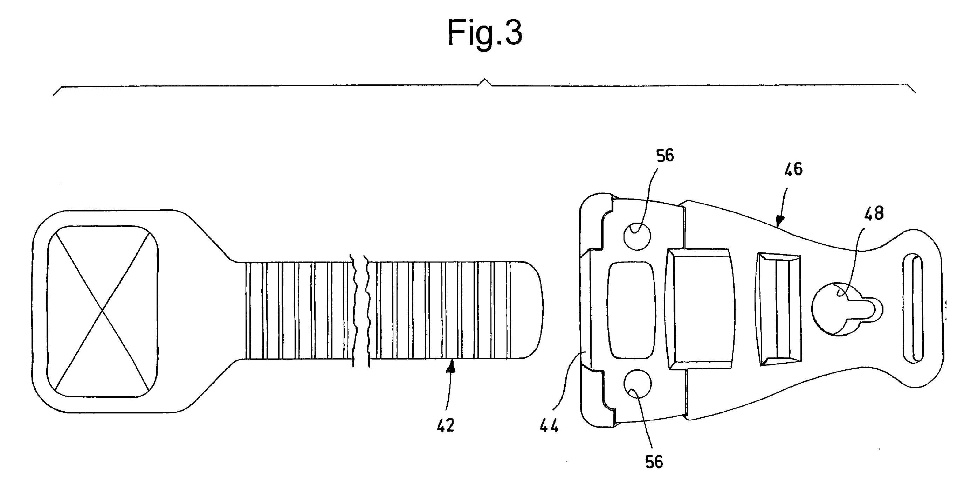

figure 3 is an exploded view of some components of the hyperextension orthopaedic corset of figure 1;

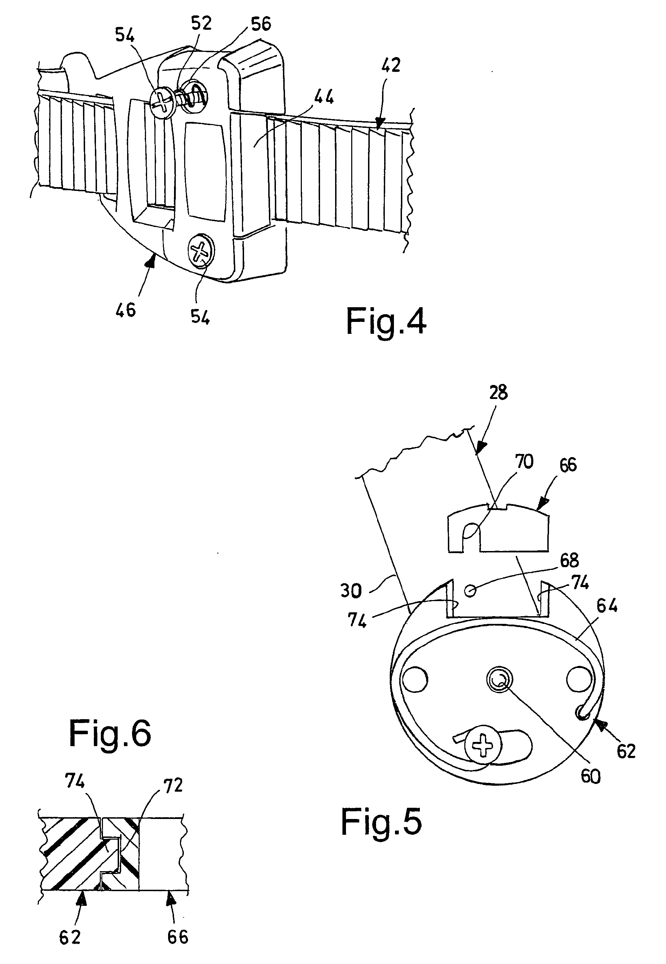

figure 4 is another view of the components of the hyperextension orthopaedic corset shown in figure 3;
figure 5 is an exploded view of further components of the hyperextension orthopaedic corset of figure 1; and
figure 6 is an enlarged detailed view of the components of the hyperextension orthopaedic corset shown in figure 5.

With reference to the figures, an improved hyperextension orthopaedic corset is shown according to the present invention, wholly indicated with reference number 10. The corset 10 consists of a frame structure in light aluminium alloy, provided with relative movements for adjusting the thoracic width, the height of the trunk and the positioning on the pelvis. Accessories which form stuffed surfaces at the contact points with the trunk of the patient, and a system of belts for tensioning the corset 10 once it is worn are applied to the frame structure, through suitable joint elements, as better specified below.

The frame structure consists of rods developing substantially horizontally, arranged at the breastbone and at the pelvis, spaced by substantially vertical lateral uprights. In detail, the frame structure comprises, at the top and in a front position with reference to the body of the user, a pair of sternal rods 12 which have a rectilinear portion 14 and a curved portion 16, so as to adapt to the shape of the chest. The end portions of the curved portions 16 comprise suitable means, not illustrated for the sake of simplicity since they are of the known type at the state of the art, for coupling with adjustable inclination with the lateral uprights 18.

Each rectilinear portion 14 of the sternal rods 12 is provided with a rectangular window 20 close to the ends and with a groove 22 which is placed on the remaining rectilinear portion. The two rectilinear portions 14 are mounted on top of each other in a position symmetrical with respect to a sagittal plane A, so as to permit a translation relative movement.

The two sternal rods 12 are kept coupled by constraining elements, which consist of two adjustment clamps 24 which engage each with the rectangular window 20 and with the groove 22, simultaneously. The adjustment clamps 24 are of the known type at the state of the art and shall not therefore be described in detail.

Two upper lateral uprights 26, right and left, are applied to the sternal rods 12 preferably but not necessarily with an angular adjustment which can be snap adjusted according to a reasonable number of positions. The connection between the sternal rods 12 and the upper lateral uprights 26 is carried out by means of threaded fixing means which also permits the aforementioned angular adjustment.

Each upper lateral upright 26 is also coupled, in a manner such as to permit the height adjustment, with a respective lower lateral upright 28, so as to form the entire lateral upright 18 of the corset 10. Each upper pair of lateral uprights 26 and lower pair of lateral uprights 28 is kept in contact through threaded constraining means, which engage into proper holes or grooves provided in the upper 26 and lower 28 lateral uprights themselves. Such constraining elements can thus be blocked in position, once the correct height adjustment of the entire lateral upright 18 has been obtained, through a suitable locking tool.

The lower lateral uprights 28 end with a lower curved portion 30, which follows the widening of the pelvis of the user and ends in turn with a joining element 32 which is articulated to a pelvic strap 34, which constitutes the lower horizontal rod for connecting the lateral uprights 18.

The tensioning system comprises a rear belt 36 whereon a lumbar plate 38 is inserted. Such a lumbar plate 38, suitably reinforced and stuffed, constitutes the rear thrust point of the corset 10. The rear belt 36 is constrainable, at its opposite ends, to the lateral uprights 18 and comprises toothed means for step-by-step adjusting the length of the rear belt 36 itself and for tensioning the corset 10.

In detail, a first end 36a of the rear belt 36 is made integral with a closing lever 40, constrained to one of the lateral uprights 18, whereas on the second end 36b of such a rear belt 36 a toothed belt 42 is obtained selectively engageable with one or more teeth 44 obtained on a hooking means 46, to the opposite lateral upright 18. The hooking means 46 is provided with a tapered hole 48, so as to allow inserting into its inside and blocking in position a projecting pin element 50 integral with one of the lateral uprights 18, hence realizing a dismountable connection of the rear belt 36 to the frame structure of the corset 10.

The tooth 44 of the hooking means 46 is provided with elastic elements 52 actuatable so as to obtain a disengaging movement between such a tooth 44 and the teeth of the toothed belt 42 and, thus, the relative sliding between the hooking means 46 and the toothed belt 42. The elastic elements 52 preferably consist of coil springs. Once the tension of the belt 36 has been correctly adjusted, through the aforementioned relative sliding between the hooking means 46 and the toothed belt 42 obtained on the second end 36b of such a belt 36, it is possible to block the movement of the tooth 44 through threaded tightening means 54 which compress the elastic elements 52. Preferably, the threaded tightening means 54 consist of screws which insert into the respective coil springs 52 and engage within threaded holes 56 obtained in the hooking means 46.

The pelvic strap 34 consists of a metal core covered by a padding 58 and curved according to an anatomical shape so as to adapt to the pelvis of the user of the corset 10. On the curved end portion 30 of the lower lateral upright a threaded pin 60 is applied, around which the plate 62 can rotate. The plate 62 is in turn rigidly constrained to the ends of the pelvic strap 34 through an anchoring system realized, for example, through screws. On the articulated joining element 32 a torsion spring 64 is applied, constrained to the pelvic strap 34 on one side and to the curved end portion 30 of the lower lateral upright 28 on the other side.

The pelvic strap 34 is provided with the possibility of oscillating with respect to the lateral uprights 18, so as to be capable of complying with the movements of the user of the corset 10. For example, when the user sits down, the pelvic strap 34 can rotate upwards so as not to hit against the legs and thus avoid the translation of the entire corset 10.

In order to block the oscillation of the pelvic strap 34, as well as to arrange it according to a predefined angle with respect to the lateral uprights 18, at least one extractable adjusting block 66 is provided, capable of cooperating with a pin 68 integral with the curved end portion 30 of the lower lateral upright 28 and with the plate 62. In detail, the adjusting block 66 is provided with one or more slots 70 for engaging with the pin 68 and with one or more grooved guides 72 for engaging with corresponding guides 74 obtained in the plate 62. Once it has been inserted into the plate 62, the adjusting block 66 is thus capable of blocking the relative rotation of the pelvic strap 34 with respect to the lateral uprights 18 of the corset 10. The plate 62 and the elements correlated with it, in particular the torsion spring 64 and the adjusting block 66, are protected by a plastic cover kept in position by a screw.

Advantageously, the inner core of the pelvic strap 34 can be manufactured with a material with variable elasticity. More precisely, the elasticity of the inner core is greater at the ends of the pelvic strap 34, so as to be able to absorb possible deformations caused during the adjusting steps of the corset 10.

Alternatively or in addition, the inner core of the pelvic strap 34 can be realized by coupling, for example through rivets 76, two metal materials having different elasticity. The metal material with greater elasticity with respect to the metal material used for the central portion of the pelvic strap 34 will thus be arranged at the ends of the pelvic strap 34.

A plate 78 for supporting the breastbone, preferably in aluminium, stuffed and suitably shaped with an anatomical shape, which is capable of an oscillating movement for adapting to the inclination of the wall of the breastbone, is applied to the sternal rods 12.

Respective stuffed lateral plates 80 are, on the other hand, applied to the lateral uprights 18, said lateral plates 80 also being anatomical shaped so as to best adapt to the hips and protect them from contact with the lateral uprights 18 themselves. Both the sternal plate 78 and the lateral plates 80 are provided with suitable means for fastening, preferably buttons, to the respective components of the aluminium structure.

The operations for applying the hyperextension corset 10 consist in buttoning the hooking means 46 of the rear belt 36 to the projecting pin element 50, so that such a rear belt 36 be adherent to the body, but not tight, and that the closing lever 40 be in the open position. After having suitably adjusted the toothed belt 42 on the relative hooking means 46, by actuating the closing lever 40 it is finally possible to tighten the rear belt 36 and to obtain the correct application of the hyperextension corset 10.

The tightening of the screws 54, along with the actuation of a safety mechanism (which is not shown) foreseen on the closing lever 40, prevents the rear belt 36 from accidentally loosening and therefore a possible complete release of the corset 10, which would cause a sudden collapsing of the trunk.

All further adjustments of the corset 10, amongst which for example the adjustment of the sternal rods 12 based upon the width of the chest and the adjustment of the lateral uprights 18 based upon the height of the trunk of the user, are carried out in a manner that is *per se* known by a specialised orthopaedics technician.

The pelvic strap 34 can, on the other hand, remain free to oscillate, counteracting the action of the torsion spring 64, or it can be blocked according to a predefined position with respect to the lateral uprights 18, as described previously.

Also in this case, in order to avoid undesired movements of the pelvic strap 34 with respect to the lateral uprights 18, the orthopaedics technician will be able to protect all internal components of the articulated joining element 32 from tampering, through the aforementioned plastic cover, kept in position by the relative screw.

It has thus been seen that the improved hyperextension orthopaedic corset according to the present invention achieves the purposes previously highlighted.

The improved hyperextension orthopaedic corset of the present invention thus conceived can in any case undergo numerous modifications and variants, all covered by the same inventive concept; moreover, all the details can be replaced by technically equivalent elements. In practice the materials used, as well as shapes and sizes, may be any according to the technical needs.

The scope of protection of the invention is thus defined by the attached claims.

## Claims

1. Hyperextension orthopaedic corset (10) constituted by a frame structure consisting of a pair of upper sternal rods (12) developing substantially horizontally, two pairs of lateral uprights (18) developing substantially vertically and a lower pelvic strap (34), substantially horizontal, for the connection between said pairs of lateral uprights (18), wherein said pair of sternal rods (12) and said pairs of lateral uprights (18) are provided with adjustable translation relative movement and are preferably provided with mutual connecting means with adjustable inclination, the corset (10) further comprising a rear belt (36) for the application to the trunk, constrainable at its opposite ends (36a, 36b) to said pairs of lateral uprights (18), and a plurality of accessories (38, 78, 80), adjustably constrainable to the frame structure, which form stuffed surfaces at the contact points of the corset (10) with the trunk, **characterised in that** said rear belt (36) comprises toothed means (42, 46) for step-by-step adjusting of length of said rear belt (36) and for tensioning the corset (10), said toothed means (42, 46) comprising a toothed belt (42), obtained on one of the two ends (36b) of the rear belt (36), and a hooking means (46), provided with at least one tooth (44) selectively engageable with said toothed belt (42) and suitable for hooking said rear belt (36) to one of said pairs of lateral uprights (18), wherein said hooking means (46) is provided with a tapered hole (48), so as to allow inserting into its inside and blocking in position a projecting pin element (50) integral with one of said pairs of lateral uprights (18), hence realizing a dismountable connection of the rear belt (36) to the frame structure of the corset (10).

2. Hyperextension orthopaedic corset (10) according to claim 1, **characterised in that** the tooth (44) of said hooking means (46) is provided with elastic elements (52) actuatable to obtain a disengagement movement between said tooth (44) and the teeth of the toothed belt (42) and, thus, a relative sliding between said hooking means (46) and said toothed belt (42).

3. Hyperextension orthopaedic corset (10) according to claim 2, **characterised in that** said hooking means (46) comprises threaded tightening means (54) capable of compressing said elastic elements (52) to block the movement of the tooth (44).

4. Hyperextension orthopaedic corset (10) according to any one of the claims from 1 to 3, **characterised in that** said pairs of lateral uprights (18) are constrained to the lower pelvic strap (34), at its ends, through a rotary joining element (32) comprising a pin (60) whereon a plate (62) provided with a torsion spring (64) rotates, constrained to the lower pelvic strap (34) on one side and to each of said pairs of lateral uprights (18) on the other side, to allow the oscillation of said lower pelvic strap (34) with respect to said pairs of lateral uprights (18).

5. Hyperextension orthopaedic corset (10) according to claim 4, **characterised in that** said rotary joining element (32) comprises at least one extractable adjusting block (66), capable of cooperating with a pin (68) integral with the lower portion (30) of each of said pairs of lateral uprights (18) and with the plate (62) for blocking the oscillation of the lower pelvic strap (34), as well as for arranging said lower pelvic strap (34) according to a predefined angle with respect to said pairs of lateral uprights (18).

6. Hyperextension orthopaedic corset (10) according to claim 5, **characterised in that** said adjusting block (66) is provided with one or more slots (70) for engaging with said pin (68) and with one or more grooved guides (72) for engaging with corresponding guides (74) obtained in the plate (62) so that, once inserted into said plate (62), the adjusting block (66) is capable of blocking the relative rotation of the lower pelvic strap (34) with respect to said pairs of lateral uprights (18).

7. Hyperextension orthopaedic corset (10) according to any of the preceding claims, **characterised in that** the lower pelvic strap (34) is provided with an inner core manufactured with a material with variable elasticity, with greater elasticity at the ends of said lower pelvic strap (34).

8. Hyperextension orthopaedic corset (10) according to any of the preceding claims, **characterised in that** the inner core of the lower pelvic strap (34) is realized by coupling two metal materials having different elasticity.

## Patentansprüche

1. Orthopädisches Hyperextensionskorsett (10), das von einer Rahmenstruktur gebildet wird, die aus einem Paar von im Wesentlichen waagrecht verlaufenden oberen Sternalstäben (12), zwei Paaren vom im Wesentlichen senkrecht verlaufenden Seitenstreben (18) und einem im Wesentlichen waagrechten unteren Beckengurt (34) für die Verbindung zwischen den Seitenstrebenpaaren (18) besteht, wobei das Paar Sternalstäbe (12) und die Seitenstrebenpaare (18) mit einer einstellbaren Translationsrelativbewegung versehen sind und vorzugsweise mit Mitteln für die gegenseitige Verbindung mit einstellbarer Neigung versehen sind, wobei das Korsett (10) ferner einen Rückengurt (36) für die Anbringung am Rumpf, der an seinen entgegengesetzten Enden (36a, 36b) an den Seitenstrebenpaaren (18) befestigt werden kann, und eine Vielzahl von Zubehörteilen (38, 78, 80) umfasst, die einstellbar an der Rahmenstruktur befestigt werden können und gepolsterte Flächen an den Berührungspunkten des Korsetts (10) mit dem Rumpf bilden, **dadurch gekennzeichnet**, **dass** der Rückengurt (36) gezahnte Mittel (42, 46) zum stufenweisen Einstellen der Länge des Rückengurts (36) und zum Spannen des Korsetts (10) umfasst, wobei diese gezahnten Mittel (42, 46) einen gezahnten Gurt (42), der an einem der zwei Enden (36b) des Rückengurts (36) ausgebildet ist, und ein Einhakmittel (46) umfassen, das mit mindestens einem Zahn (44) versehen ist, der selektiv mit dem gezahnten Gurt (42) in Eingriff gebracht werden kann und geeignet ist, um den Rückengurt (36) mit einem der Seitenstrebenpaare (18) zu verhaken, wobei dieses Einhakmittel (46) mit einem konischen Loch (48) versehen ist, um zu ermöglichen, ein vorstehendes Stiftelement (50), das integral mit einem von den Seitenstrebenpaaren (18) ist, in sein Inneres einzuführen und zu arretieren und so eine lösbare Verbindung des Rückengurts (36) mit der Rahmenstruktur des Korsetts (10) zu schaffen.

2. Orthopädisches Hyperextensionskorsett (10) nach Anspruch 1, **dadurch gekennzeichnet, dass** der Zahn (44) des Einhakmittels (46) mit elastischen Elementen (52) versehen ist, die betätigt werden können, um eine Ausrastbewegung zwischen dem Zahn (44) und den Zähnen des gezahnten Gurts (42) und somit ein relatives Gleiten zwischen dem Einhakmittel (46) und dem gezahnten Gurt (42) zu erhalten.

3. Orthopädisches Hyperextensionskorsett (10) nach Anspruch 2, **dadurch gekennzeichnet, dass** das Einhakmittel (46) Gewindespannmittel (54) umfasst, welche die elastischen Elemente (52) zusammendrücken können, um die Bewegung des Zahns (44) zu blockieren.

4. Orthopädisches Hyperextensionskorsett (10) nach einem der Ansprüche von 1 bis 3, **dadurch gekennzeichnet, dass** die Seitenstrebenpaare (18) am unteren Beckengurt (34) an seinen Enden durch ein Drehverbindungselement (32), das einen Zapfen (60) umfasst, auf dem sich eine mit einer Torsionsfeder (64) versehene Platte (62) dreht, befestigt sind, das auf der einen Seite am unteren Beckengurt (34) und auf der anderen Seite an jedem der Seitenstrebenpaare (18) befestigt ist, um das Schwingen des unteren Beckengurts (34) gegenüber den Seitenstrebenpaaren (18) zu ermöglichen.

5. Orthopädisches Hyperextensionskorsett (10) nach Anspruch 4, **dadurch gekennzeichnet, dass** das Drehverbindungselement (32) mindestens einen ausziehbaren Verstellblock (66) umfasst, der mit einem Zapfen (68), der integral mit dem unteren Abschnitt (30) von jedem der Seitenstrebenpaare (18) ist, und mit der Platte (62) zusammenwirken kann, um das Schwingen des unteren Beckengurts (34) zu blockieren und auch um den unteren Beckengurt (34) in einem vorbestimmten Winkel zu den Seitenstrebenpaaren (18) anzuordnen.

6. Orthopädisches Hyperextensionskorsett (10) nach Anspruch 5, **dadurch gekennzeichnet, dass** der Verstellblock (66) mit einem oder mehreren Schlitzen (70) für den Eingriff mit dem Zapfen (68) und mit einer oder mehreren nutförmigen Führungen (72) für den Eingriff mit in der Platte (62) ausgebildeten entsprechenden Führungen (74) versehen ist, so dass der Verstellblock (66), wenn er in die Platte (62) eingefügt ist, die Relativdrehung des unteren Beckengurts (34) gegenüber den Seitenstrebenpaaren (18) blockieren kann.

7. Orthopädisches Hyperextensionskorsett (10) nach einem der vorhergehenden Ansprüche, **dadurch gekennzeichnet, dass** der untere Beckengurt (34) mit einem aus einem Material mit variabler Elastizität hergestellten Innenkern mit einer größeren Elastizität an den Enden des unteren Beckengurts (34) versehen ist.

8. Orthopädisches Hyperextensionskorsett (10) nach einem der vorhergehenden Ansprüche, **dadurch gekennzeichnet, dass** der Innenkern des unteren Beckengurts (34) durch Verbinden von zwei metallischen Werkstoffen mit unterschiedlicher Elastizität realisiert ist.

## Revendications

1. Corset orthopédique d'hyperextension (10) constitué par une structure de support comprenant une paire de tiges sternales supérieures (12) s'étendant sensiblement horizontalement, deux paires de montants latéraux (18) s'étendant sensiblement verticalement et une sangle pelvienne inférieure (34), sensiblement horizontale, pour la connexion entre lesdites paires de montants latéraux (18), dans lequel ladite paire de tiges sternales (12) et lesdites paires de montants latéraux (18) sont dotées d'un mouvement relatif de translation réglable et sont munies de préférence de moyens de connexion mutuelle avec inclinaison réglable, le corset (10) comprenant en outre une courroie postérieure (36) pour l'application au tronc, pouvant être fixée à ses extrémités opposées (36a, 36b) auxdites paires de montants latéraux (18), et une pluralité d'accessoires (38, 78, 80), pouvant être fixés à la structure de support, qui forment des surfaces rembourrées au niveau des points de contact du corset (10) avec le tronc, **caractérisé en ce que** ladite courroie postérieure (36) comprend des moyens dentés (42, 46) pour le réglage pas à pas de la longueur de ladite courroie postérieure (36) et pour la mise en tension du corset (10), lesdits moyens dentés (42, 46) comprenant une courroie dentée (42), obtenue sur une des deux extrémités (36b) de la courroie postérieure (36), et un moyen d'accrochage (46), muni d'au moins une dent (44) pouvant être engagée sélectivement avec ladite courroie dentée (42) et adaptée pour accrocher ladite courroie postérieure (36) à une desdites paires de montants latéraux (18), dans lequel ledit moyen d'accrochage (46) est pourvu d'un trou conique (48), de manière à permettre l'insertion à l'intérieur de celui-ci et le blocage en position d'un élément de broche en saillie (50) d'une seule pièce avec une desdites paires de montants latéraux (18), en réalisant ainsi une connexion démontable de la courroie postérieure (36) avec la structure de support du corset (10).

2. Corset orthopédique d'hyperextension (10) selon la revendication 1, **caractérisé en ce que** la dent (44) dudit moyen d'accrochage (46) est munie d'éléments élastiques (52) actionnables pour obtenir un mouvement de désengagement entre ladite dent (44) et les dents de la courroie dentée (42) et, ainsi, un coulissement relatif entre ledit moyen d'accrochage (46) et ladite courroie dentée (42).

3. Corset orthopédique d'hyperextension (10) selon la revendication 2, **caractérisé en ce que** ledit moyen d'accrochage (46) comprend des moyens de serrage filetés (54) capables de comprimer lesdits éléments élastiques (52) pour bloquer le mouvement de la dent (44).

4. Corset orthopédique d'hyperextension (10) selon l'une quelconque des revendications 1 à 3, **caractérisé en ce que** lesdites paires de montants latéraux (18) sont fixées à la sangle pelvienne inférieure (34), à ses extrémités, par l'intermédiaire d'un élément de jonction rotative (32) comprenant une broche (60) sur laquelle tourne une plaque (62) munie d'un ressort de torsion (64), fixé à la sangle pelvienne inférieure (34) d'un côté et à chacune desdites paires de montants latéraux (18) de l'autre côté, pour permettre l'oscillation de ladite sangle pelvienne inférieure (34) par rapport auxdites paires de montants latéraux (18).

5. Corset orthopédique d'hyperextension (10) selon la revendication 4, **caractérisé en ce que** ledit élément de jonction rotative (32) comprend au moins un bloc de réglage extractible (66), capable de coopérer avec une broche (68) d'une seule pièce avec la portion inférieure (30) de chacune desdites paires de montants latéraux (18) et avec la plaque (62) pour bloquer l'oscillation de la sangle pelvienne inférieure (34), ainsi que pour disposer ladite sangle pelvienne inférieure (34) suivant un angle prédéfini par rapport auxdites paires de montants latéraux (18).

6. Corset orthopédique d'hyperextension (10) selon la revendication 5, **caractérisé en ce que** ledit bloc de réglage (66) est pourvu d'une ou plusieurs fentes (70) pour l'engagement avec ladite broche (68) et avec un ou plusieurs guides rainurés (72) pour l'engagement avec des guides correspondants (74) obtenus dans la plaque (62) de manière que, une fois inséré dans ladite plaque (62), le bloc de réglage (66) soit capable de bloquer la rotation relative de la sangle pelvienne inférieure (34) par rapport auxdites paires de montants latéraux (18).

7. Corset orthopédique d'hyperextension (10) selon l'une quelconque des revendications précédentes, **caractérisé en ce que** la sangle pelvienne inférieure (34) est munie d'une âme interne fabriquée avec un matériau avec élasticité variable, avec une élasticité supérieure aux extrémités de ladite sangle pelvienne inférieure (34).

8. Corset orthopédique d'hyperextension (10) selon l'une quelconque des revendications précédentes, **caractérisé en ce que** l'âme interne de la sangle pelvienne inférieure (34) est réalisée en couplant deux matériaux métalliques ayant une élasticité différente.
